(19) Europäisches Patentamt
European Patent Office
Office européen des brevets

(11) Veröffentlichungsnummer: **0 378 080**
**A1**

(12) # EUROPÄISCHE PATENTANMELDUNG

(21) Anmeldenummer: **90100055.4**

(22) Anmeldetag: **03.01.90**

(51) Int. Cl.5: **C07C 69/743, A01N 53/00, C07C 67/08**

(30) Priorität: **07.01.89 DE 3900275**

(43) Veröffentlichungstag der Anmeldung:
**18.07.90 Patentblatt 90/29**

(84) Benannte Vertragsstaaten:
**BE CH DE DK ES FR GB IT LI NL**

(71) Anmelder: **BASF Aktiengesellschaft**
**Carl-Bosch-Strasse 38**
**D-6700 Ludwigshafen(DE)**

(72) Erfinder: **Theobald, Hans, Dr.**
**Queichstrasse 6**
**D-6703 Limburgerhof(DE)**
Erfinder: **Wild, Jochen, Dr.**
**An der Marlach 7**
**D-6705 Deidesheim(DE)**
Erfinder: **Wolf, Bernd, Dr.**
**Hallbergstrasse 4**
**D-6701 Fussgoenheim(DE)**
Erfinder: **Zombik, Winfried, Dr.**
**Kallstadter Strasse 4**
**D-6804 Ilvesheim(DE)**
Erfinder: **Kuenast, Christoph, Dr.**
**Salierstrasse 2**
**D-6701 Otterstadt(DE)**
Erfinder: **Hofmeister, Peter, Dr.**
**Bernard-Humblot-Strasse 12**
**D-6730 Neustadt(DE)**

(54) **Substituierte Cyclopropancarbonsäurepropargylester, Verfahren zu ihrer Herstellung und ihre Verwendung zur Bekämpfung von Schädlingen.**

(57) Die vorliegende Erfindung betrifft neue substituierte Cyclopropancarbonsäurepropargylester der allgemeinen Formel I

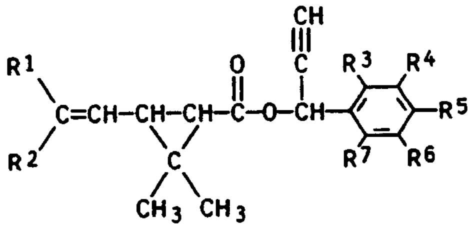

I,

in der die Substituenten die folgende Bedeutung haben:
$R^1$, $R^2$ $C_1$-$C_4$-Halogenalkyl oder Halogen, wobei nicht beide Reste gleichzeitig für Halogen stehen,
$R^3$ bis $R^7$ unabhängig voneinander Wasserstoff, Halogen, Cyano, $C_1$-$C_6$-Alkyl, $C_2$-$C_6$-Alkenyl, $C_2$-$C_6$-Alkinyl, $C_1$-$C_6$-Alkoxy, wobei die Kohlenwasserstoffreste jeweils unsubstituiert oder durch Halogen substituiert sind, oder jeweils zwei benachbarte Reste über eine Gruppe -O-X-O- verbunden sind, wobei
X eine gegebenenfalls halogensubstituierte Methylen- oder Ethylenkette bedeutet,
mit der Maßgabe, daß $R^4$ nicht Trifluormethyl bedeutet, wenn $R^5$ für ein Chloratom steht, sowie deren Herstellung und Verwendung zur Bekämpfung von Schädlingen.

EP 0 378 080 A1

## Substituierte Cyclopropancarbonsäurepropargylester, Verfahren zu ihrer Herstellung und ihre Verwendung zur Bekämpfung von Schädlingen

Die vorliegende Erfindung betrifft neue substituierte Cyclopropancarbonsäurepropargylester der allgemeinen Formel I

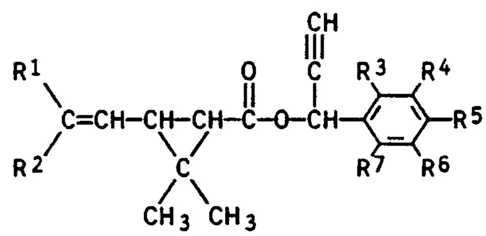

I,

in der die Substituenten die folgende Bedeutung haben:
$R^1$, $R^2$ $C_1$-$C_4$-Halogenalkyl oder Halogen, wobei nicht beide Reste gleichzeitig für Halogen stehen,
$R^3$ bis $R^7$ unabhängig voneinander Wasserstoff, Halogen, Cyano, $C_1$-$C_6$-Alkyl, $C_2$-$C_6$-Alkenyl, $C_2$-$C_6$-Alkinyl, $C_1$-$C_6$-Alkoxy, wobei die Kohlenwasserstoffreste jeweils unsubstituiert oder durch Halogen substituiert sind, oder jeweils zwei benachbarte Reste über eine Gruppe -O-X-O- verbunden sind, wobei
X eine gegebenenfalls halogensubstituierte Methylen- oder Ethylengruppe bedeutet,
mit der Maßgabe, daß $R^4$ nicht Trifluormethyl bedeutet, wenn $R^5$ für ein Chloratom steht.

Ferner betrifft die Erfindung ein Verfahren zur Herstellung der Verbindungen I, Schädlingsbekämpfungsmittel, die diese Verbindungen als Wirkstoffe enthalten sowie Verfahren zur Bekämpfung von Schädlingen mit diesen Wirkstoffen.

Aus den japanischen Offenlegungsschriften 144 349/1983, 121 246/1983 und 095 940/1982 ist bekannt, daß speziell substituierte Cyclopropancarbonsäurepropargylester eine insektizide Wirkung zeigen. In der DE-A 31 45 448 werden 2,2-Dihalogenvinyl-substituierte Cyclopropancarbonsäurepropargylester zur Bekämpfung von Schädlingen offenbart. Die insektizide Wirksamkeit der in der Literatur beschriebenen Ester ist jedoch unter bestimmten Bedingungen, z.B. niedrigen Aufwandmengen, in den meisten Fällen unbefriedigend.

Der Erfindung lag daher die Aufgabe zugrunde, neue substituierte Cyclopropancarbonsäurepropargylester mit verbesserter Wirkung bereitzustellen.

Demgemäß wurden die eingangs definierten Cyclopropancarbonsäurepropargylester sowie Verfahren zu deren Herstellung gefunden. Ferner wurde gefunden, daß sich die Verbindungen I hervorragend zur Bekämpfung von Schädlingen eignen.

Die Ester der Formel I können durch Umsetzung der Säuren II oder Derivaten dieser Säuren mit α-Ethinylalkoholen III nach folgendem allgemeinen Reaktionsschema gewonnen werden:

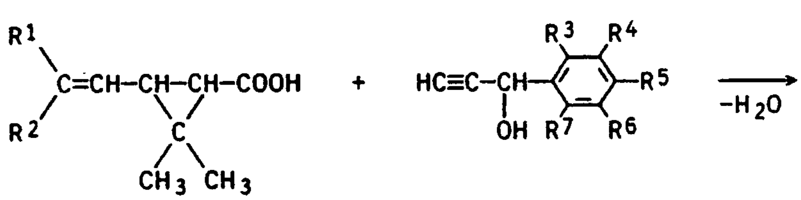


I

II III

Die Umsetzung kann in an sich bekannter Weise durch Zusatz von Katalysatoren wie Schwefelsäure, Halogenwasserstoff, Sulfonsäuren, sauren Ionenaustauschern beschleunigt und das Veresterungsgleichgewicht im gewünschten Sinne verschoben werden, indem man dem Reaktionsgemisch das Wasser oder den Ester I entzieht, z.B. durch azeotrope Destillation oder durch Bindung des Wassers an Schwefel- oder Halogenwasserstoffsäure.

Ebensogut können reaktive Carbonsäurederivate wie die entsprechenden Säurehalogenide, z.B. Säurechloride, mit den Alkoholen der Formel III in Gegenwart eines Säureakzeptors umgesetzt werden (vgl. Houben-Weyl, Methoden der Organ. Chemie, Band VIII, S. 541 ff, Georg-Thieme-Verlag, Stuttgart 1952).

Als Säurebindemittel eignen sich die üblichen basischen Mittel, insbesondere aliphatische, aromatische

und heterocyclische Amine, z.B. Triethylamin, Dimethylamin, Piperidin, Dimethylanilin, Dimethylbenzylamin, Pyridin und 2-Picolin.

Die Umsetzung kann in einem Lösungs- oder Verdünnungsmittel ausgeführt werden. Hierzu sind die angeführten Säureakzeptoren selbst oder beispielsweise folgende Lösungs- oder Verdünnungsmittel oder Gemische derselben geeignet:
Aliphatische und aromatische, gegebenenfalls chlorierte Kohlenwasserstoffe wie Petrolether, Benzol, Toluol, Xylol, Benzin, Dichlormethan, Chloroform, Tetrachlormethan, 1,2-Dichlorethan, Chlorbenzol; Ether wie Diethyl- und Di-n-butylether, Methyl-tert.-butylether, Tetrahydrofuran, Dioxan; Ketone, beispielsweise Aceton, Methylethylketon, Methylisopropylketon; ferner Nitrile wie Acetonitril und Propionitril.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung der Säurehalogenide verläuft gewöhnlich oberhalb von 0°C mit ausreichender Geschwindigkeit. Da sie meist unter Wärmeentwicklung verläuft, kann es von Vorteil sein, eine Kühlmöglichkeit vorzusehen.

Die erfindungsgemäßen Ester können außerdem noch nach praktisch allen bekannten Verfahren der Estersynthese hergestellt werden, beispielsweise durch Umsetzung von entsprechenden Säureanhydriden mit den Alkoholen der Formel III, durch Umsetzung von entsprechenden Salzen mit Derivaten der Alkohole der Formel III oder durch Umesterung (vgl. Houben-Weyl a.a.O., S. 508-628).

Die als Ausgangsprodukte benötigten Säurekomponenten der allgemeinen Formel II und ihre Derivate sind literaturbekannt, z.B. aus Wegler, Chemie der Pflanzenschutz- und Schädlingsbekämpfungsmittel, Band 7, Springer-Verlag, Berlin, Heidelberg, New York 1981.

Die als Ausgangsprodukte benötigten Alkohole der Formel III können durch Umsetzung der entsprechenden Aldehyde mit metallorganischen Verbindungen des Acetylens, z.B. Grignardverbindungen oder Lithiumorganylverbindungen, hergestellt werden.

Die Umsetzungen mit den Metallorganylen kann in an sich bekannter Weise, z.B. wie in Houben-Weyl, Methoden der organischen Chemie, Band 13/2a, S. 285ff, 1973 beschrieben, in einem inerten organischen Lösungsmittel wie Ether oder Tetrahydrofuran unter Schutzgas durchgeführt werden, so daß sich nähere Ausführungen hierzu erübrigen.

Es versteht sich, daß die Verbindungen der Formel I in jedem Falle in Form von einheitlichen Diastereomeren, mindestens einem Paar otpischer Antipoden, in vielen Fällen auch in Form von Diastereomeren-Vielfachen vorkommen und als Wirkstoffe angewandt werden können, die je nach den Ausgangsstoffen und den Umsetzungsbedingungen in einheitlicher Form oder als Gemische auftreten. Die Gemische können in üblicher Weise in ihre sterisch einheitlichen Bestandteile aufgetrennt werden; ihre biologische Wirkung ist in Einzelfällen von ihrer sterischen Konfiguration abhängig.

In den erfindungsgemäßen Verbindungen I haben die Reste $R^1$ bis $R^7$ im einzelnen die Bedeutung:
$R^1$, $R^2$ $C_1$-$C_4$-Halogenalkyl, bevorzugt $C_1$-$C_4$-Fluor- und Chloralkyl wie Trifluormethyl, Difluormethyl, Fluormethyl, Trichlormethyl, Pentafluorethyl, 2,2,2-Trifluoreth-1-yl, 2,2,2-Trichloreth-1-yl, 1-Fluormethyl-(2-fluor)-ethyl und 1-Chlormethyl-(2-chlor)-ethyl, wobei Trifluormethyl besonders bevorzugt ist,
Halogen wie Fluor, Chlor oder Brom;
$R^3$ bis $R^7$ Wasserstoff;
Halogen wie Fluor, Chlor, Brom oder Jod;
Cyano;
$C_1$-$C_6$-Alkyl, insbesondere $C_1$-$C_4$-Alkyl wie Methyl, Ethyl, Propyl, iso-Propyl, Butyl, iso-Butyl, sec.-Butyl, tert.-Butyl, wobei die genannten Reste durch ein oder mehrere, insbesondere 1 bis 4 Halogenatome, z.B. Chlor oder Fluor, substituiert sein können;
$C_2$-$C_6$-Alkenyl wie Vinyl, Allyl, 1-Methylallyl, 1,3-Dimethyl-but-2-enyl, 1-Methyl-but-2-enyl oder But-3-en-1-yl, wobei die genannten Reste durch ein oder mehrere, insbesondere 1 bis 4 Halogenatome, z.B. Chlor oder Fluor, substituiert sein können;
$C_2$-$C_6$-Alkinyl wie Ethinyl, Prop-1-in-1-yl, Prop-2-in-1-yl, 1-Methyl-prop-2-inyl, But-2-in-2-yl und But-3-in-1-yl, wobei die genannten Reste durch 1 oder mehrere, insbesondere 1 bis 4 Halogenatome, z.B. Chlor oder Fluor, substituiert sein können;
$C_1$-$C_6$-Alkoxy wie Methoxy, Ethoxy, Propoxy, Isopropoxy, Butoxy, tert.-Butoxy, wobei die Alkylgruppen durch ein oder mehrere, insbesondere 1 bis 4 Halogenatome, z.B. Chlor oder Fluor substituiert sein können;
zwei benachbarte Reste $R^3$ und $R^4$, $R^4$ und $R^5$, $R^5$ und $R^6$ oder $R^6$ und $R^7$ können auch jeweils über eine Gruppe O-X-O miteinander verbunden sein, wobei
X eine gegebenenfalls halogensubstituierte Methylen oder Ethylengruppe bedeutet und als Halogensubstituenten insbesondere Fluor oder Chlor in Betracht kommen.

Besonders bevorzugt sind Verbindungen I, in denen $R^1$ Trifluormethyl und $R^2$ Fluor, Chlor oder Brom

bedeuten und der Phenylrest unsubstituiert ist oder 1 bis 3 der o.g. Substituenten trägt.

Die Cyclopropancarbonsäurepropargylester der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Ortiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Geradflügler (Orthoptera) beispielsweise Forficula auricularia, Acheta Domestica, Gryllotalpa gryllotalpa, Tachycines asynamorus, Locusta migratoria, Stauronotus maroccanus, Schistocerca peregrina, Nomadacris septemfasciata, Melanoplus spretus, Melanoplus femur-rubrum, Blatta orientalis, Blattella germanica, Periplaneta americana, Blabera gigantes.

Zur Klasse der Arachnoidea gehören Spinnentiere (Acarina) beispielsweise Ixodes ricinus, Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schachtii, Heterodera trifolii, Stock- und Blattälchen,

z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Paratylenchus neglectus, Pratylenchus penetrans, Paratylenchus curvitatus, Paratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 4 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 13 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 19 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 Gew.%, vorzugsweise zwischen 0,01 und 1 Gew.%.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,02 bis 10, vorzugsweise 0,1 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die Herstellung der erfindungsgemäßen Verbindungen kann gemäß der Beschreibung des folgenden Beispiels erfolgen:

Herstellungsbeispiel

2,2-Dimethyl-3-(3′,3′,3′-trifluor-2′-chlorpropen-(1)-yl)-cyclo-propancarbonsäure-1″-(2‴,6‴-difluorphenyl)-propin-(2″)-ester (trans-Form).

5 g (0,03 mol) 1-Hydroxy-1-(2′,6′-difluorphenyl)-propin(2) und 4 g (0,04 mol) Triethylamin werden in 100 ml Toluol gelöst und bei 15-20°C mit 7,8 g (0,03 mol) 2,2-Dimethyl-3-(2′-chlor-3′,3′,3′-trifluorpropen-(1)-)-cyclopropancarbonsäure-(1)-chlorid (trans-Form) tropfenweise versetzt. Nach Beendigung der exothermen Reaktion wird 4 Stunden bei 40°C nachgerührt. Nach dem Abkühlen wird mit Wasser gewaschen, die organische Phase getrocknet und das Lösungsmittel abgetrennt. Man erhält 11 g (93,4 % der Theorie) eines hellgelben Öls.

| CH-Analyse (Molgewicht = 392,5) | | | | |
|---|---|---|---|---|
| | C | H | Cl | F |
| Berechnet.: | 55,1 | 3,6 | 9,0 | 24,2 |
| Gefunden.: | 55,3 | 4,0 | 9,1 | 23,9 |

300 MHz-NMR-Spektrum in $CDCl_3$ (ppm):
1,24(6H); 1,83(1H); 2,42(1H); 2,62(1H); 6,14(1H); 6,79(1H); 6,86-7,0(2H); 7,37(1H).

Die in der folgenden Tabelle aufgeführten Verbindungen werden analog hergestellt:

Tabelle 1: Substituierte Cyclopropancarbonsäurepropargylester I

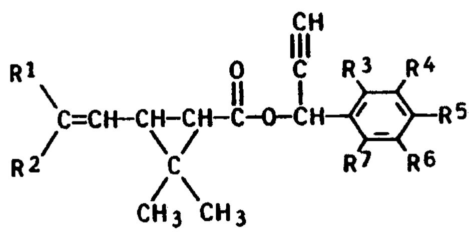

I

| Bsp. Nr. | Konfiguration der Säure | $R^1$ | $R^2$ | $R^3$ | $R^4$ | $R^5$ | $R^6$ | $R^7$ | H1-NMR-Signale a) (MHz;δ,ppm) |
|---|---|---|---|---|---|---|---|---|---|
| 2 | trans | $CF_3$ | Cl | $CH_3CH_2$ | H | H | H | H | 300; 1,82 |
| 3 | cis/trans | $CF_3$ | Cl | $CH_3$ | H | $CH_3$ | H | H | 300; 2,62 |
| 4 | trans | $CF_3$ | Cl | H | H | $(CH_3)_2CH-$ | H | H | 300; 2,91 |
| 5 | cis/trans | Cl | $CF_3$ | H | H | $(CH_3)_2CH-$ | H | H | 300; 6,12 |
| 6 | cis/trans | Cl | $CF_3$ | $CH_3CH_2$ | H | H | H | H | 300; 6,15 |
| 7 | cis/trans | Cl | $CF_3$ | H | H | $CH_3CH_2$ | H | H | 300, 2,07 |
| 8 | cis/trans | Cl | $CF_3$ | $CH_3O$ | $CH_3O$ | $CH_3O$ | H | H | 300; 3,88 |
| 9 | cis/trans | $CF_3$ | Cl | Cl | Cl | H | H | H | 250; 2,72 |
| 10 | trans | $CF_3$ | Cl | H | $ClFCHCF_2O$ | H | H | H | 200; 2,72 |
| 11 | trans | $CF_3$ | Cl | Cl | H | H | H | Cl | 200; 1,25(6H); 1,89(1H); 2,48(1H); 2,66(1H); 6,18(1H); 7,15-7,4(3H); |
| 12 | trans | $CF_3$ | Cl | H | $CH_3O$ | $F_2CHO$ | H | H | 200; 1,26(3H); 1,35(3H); 1,86(1H); 2,47(1H); 2,74(1H); 3,93(3H); |
| 13 | trans | $CF_3$ | Cl | Cl | Cl | H | H | H | 200; 6,81 |
| 14 | cis/trans | $CF_3$ | Cl | Cl | Cl | Cl | H | H | 200; 6,17 |

| Bsp. Nr. | Konfiguration der Säure | $R^1$ | $R^2$ | $R^3$ | $R^4$ | $R^5$ | $R^6$ | $R^7$ | H1-NMR-Signale a) (MHz;δ,ppm) |
|---|---|---|---|---|---|---|---|---|---|
| 15 | cis/trans | $CF_3$ | Cl | H | $CH_3O$ | $F_2CHO$ | H | H | 200; 2,7 |
| 16 | trans | Cl | $CF_3$ | Cl | Cl | Cl | H | H | 200; 6,16 |
| 17 | cis/trans | $CF_3$ | Cl | Cl | H | H | H | Cl | 200; 2,64 |
| 18 | cis/trans | Cl | $CF_3$ | H | $ClFCHCF_2O$ | H | H | H | 200; 1,17(3H); 1,29(3H); 2,72(1H); 6,88(1H); 7,25-7,5(4H) |
| 19 | trans | Cl | $CF_3$ | H | $O-CH_2-O$ | | H | H | 300; 5,97 |
| 20 | cis/trans | Cl | $CF_3$ | H | $O-CH_2-O$ | | H | H | 300; 1,22(3H); 1,25(3H); 1,92(1H); 2,3(1H); 2,68(1H) |
| 21 | trans | $CF_3$ | Cl | H | H | Br | H | H | 300; 1,81 |
| 22 | trans | Cl | $CF_3$ | H | H | H | H | $CH_3O$ | 300; 3,8 |
| 23 | cis/trans | Cl | $CF_3$ | H | H | H | H | $CH_3O$ | 300; 2,6 |
| 24 | cis/trans | Cl | $CF_3$ | H | H | CN | H | H | 300; 2,75 |
| 25 | trans | $CF_3$ | Cl | H | H | $(H_3C)_3C$ | H | H | 300; 1,32 |
| 26 | cis/trans | $CF_3$ | Cl | F | H | H | H | F | 250; 1,2(3H); 1,29(3H); 2,61(1H); 5,86(1H) |
| 27 | cis/trans | $CF_3$ | Cl | H | H | $(H_3C)_3C$ | H | H | 200; 1,31 |
| 28 | trans | Cl | $CF_3$ | H | H | CN | H | H | 300; 2,75 |
| 29 | cis/trans | Cl | $CF_3$ | H | H | Br | H | H | 300; 2,69 |
| 30 | trans | $CF_3$ | Cl | H | H | $F_2CHCF_2O$ | H | H | 300; 6,5 |
| 31 | cis/trans | Cl | $CF_3$ | H | $F_2HCO$ | H | H | H | 200; 2,71 |
| 32 | trans | Cl | $CF_3$ | H | $F_2HCO$ | H | H | H | 200; 1,16(3H); 1,26(3H); 2,68(1H); 7,12-7,5(4H) |
| 33 | cis/trans | Cl | $CF_3$ | H | H | $F_2CHCF_2O$ | H | H | 300; 1,82 |

| Bsp. Nr. | Konfiguration der Säure | $R^1$ | $R^2$ | $R^3$ | $R^4$ | $R^5$ | $R^6$ | $R^7$ | H1-NMR-Signale a) (MHz;δ,ppm) |
|---|---|---|---|---|---|---|---|---|---|
| 34 | trans | $CF_3$ | Cl | H | H | $CH_3CH_2$ | H | H | 300; 6,11 |
| 35 | trans | Cl | $CF_3$ | $CH_3$ | H | $CH_3$ | H | H | 200; 6,6 |
| 36 | trans | Cl | $CF_3$ | H | H | $CH_3O$ | $CH_3$ | $CH_3O$ | 200; 3,9 |
| 37 | cis/trans | Cl | $CF_3$ | H | H | $CH_3CH_2O$ | H | H | 200; 4,07 |
| 38 | trans | Cl | $CF_3$ | H | H | $CH_3O$ | H | H | 300; 3,82 |
| 39 | trans | $CF_3$ | Cl | H | H | $CH_3CH_2O$ | H | H | 200; 3,59 |
| 40 | cis/trans | $CF_3$ | Cl | H | H | $CH_3O$ | H | H | 300; 3,82 |
| 41 | cis/trans | Cl | $CF_3$ | H | $CF_3$ | H | H | H | 300; 1,21(3H); 1,32(3H); 2,76(1H); 6,5(1H); 7,5-7,9(4H) |
| 42 | cis/trans | Cl | $CF_3$ | H | H | H | $CH_3$ | H | 300; 2,67 |
| 43 | trans | $CF_3$ | Cl | H | H | $CF_3$ | H | H | 200; 2,77 |
| 44 | trans | $CF_3$ | Cl | H | H | H | $CF_3$ | H | 300; 2,75 |
| 45 | trans | Cl | $CF_3$ | H | H | H | $CH_3$ | H | 300; 6,14 |
| 46 | cis/trans | Cl | $CF_3$ | H | H | $CF_3$ | H | H | 300; 2,73 |
| 47 | cis/trans | Cl | $CF_3$ | H | H | H | H | $CF_3$ | 300; 6,76 |
| 48 | cis/trans | Cl | $CF_3$ | H | H | H | H | $CH_3$ | 200; 6,97 |
| 49 | trans | Cl | $CF_3$ | $CH_3$ | H | H | H | H | 300; 2,66 |
| 50 | cis/trans | $CF_3$ | Cl | H | H | $CH_3$ | H | H | 200; 2,7 |
| 51 | trans | $CF_3$ | Cl | H | H | $CH_3$ | H | H | 300; 6,46 |
| 52 | trans | Cl | $CF_3$ | H | H | H | H | $CF_3$ | 200; 6,78 |
| 53 | cis/trans | $CF_3$ | Cl | H | H | $F_2CHO$ | H | H | 300; 2,71 |
| 54 | trans | $CF_3$ | Cl | H | H | $F_2CHO$ | H | H | 300; 1,21(3H); 1,3(3H); 1,82(1H); 2,45(1H); 2,72(1H) |

| Bsp. Nr. | Konfiguration der Säure | $R^1$ | $R^2$ | $R^3$ | $R^4$ | $R^5$ | $R^6$ | $R^7$ | $H^1$-NMR-Signale [a] (MHz;δ,ppm) |
|---|---|---|---|---|---|---|---|---|---|
| 55 | trans | $CF_3$ | Cl | H | H | Cl | H | H | 300; 6,46 |
| 56 | trans | $CF_3$ | Cl | H | H | F | H | H | 300; 2,7 |
| 57 | trans | Cl | $CF_3$ | H | H | H | F | H | 300; 6,16 |
| 58 | cis/trans | Cl | $CF_3$ | Cl | H | H | H | H | 200; 6,78 |
| 59 | cis/trans | Cl | $CF_3$ | H | H | H | Cl | H | 300; 6,13 |
| 60 | cis/trans | $CF_3$ | Cl | H | H | Cl | H | H | 250; 6,46 |
| 61 | cis/trans | $CF_3$ | Cl | H | H | F | H | H | 300; 2,72 |
| 62 | cis/trans | $CF_3$ | Cl | H | F | H | H | H | 200; 6,45 |
| 63 | trans | Cl | $CF_3$ | H | H | H | H | $F_2CHCF_2O$ | 200; 2,69 |
| 64 | cis/trans | Cl | $CF_3$ | H | CN | H | H | H | 300; 1,2(3H); 1,29(3H); 2,69(1H); 6,9(1H); 7,2-7,8(4H) |
| 65 | cis/trans | Cl | $CF_3$ | Br | H | H | H | H | 300; 6,17 |
| 66 | cis/trans | Cl | $CF_3$ | H | Br | H | H | H | 200; 6,9 |
| 67 | trans | Cl | $CF_3$ | F | H | H | H | H | 300; 6,16 |
| 68 | cis/trans | Cl | $CF_3$ | H | H | H | H | F | 300; 2,7 |
| 69 | cis/trans | Cl | $CF_3$ | H | $F_2CHCF_2O$ | H | H | H | 300; 1,22(3H); 1,31(3H); 2,72(1H); 7,22(1H); 7,3-7,6(3H) |
| 70 | trans | Cl | $CF_3$ | H | $CH_3O$ | H | H | H | 200; 3,87 |
| 71 | cis/trans | Cl | $CF_3$ | H | H | H | H | $CHF_2CF_2O$ | 200; 6,76 |
| 72 | trans | Cl | $CF_3$ | H | Br | H | H | H | 300; 6,44 |
| 73 | cis/trans | $CF_3$ | Cl | H | H | H | $CH_3O$ | H | 200; 6,86 |

| Bsp. Nr. | Konfiguration der Säure | $R^1$ | $R^2$ | $R^3$ | $R^4$ | $R^5$ | $R^6$ | $R^7$ | H1-NMR-Signale [a)] (MHz;δ,ppm) |
|---|---|---|---|---|---|---|---|---|---|
| 74 | cis/trans | $CF_3$ | Cl | H | Cl | F | H | H | 300; 1,21(3H); 1,33(3H); 1,9(1H); 2,35(1H); 2,7(1H) |
| 75 | cis | $CF_3$ | Cl | H | $CF_3$ | H | H | H | |

[a)] die NMR-Spektren wurden in $CDCl_3$ als Lösungsmittel mit Tetramethylsilan als inneren Standard (δ=0 ppm) aufgenommen, ein für die jeweilige Verbindung charakteristisches Signal ist angegeben.

Anwendungsbeispiele

In den folgenden Beispielen wurden die erfindungsgemäßen Verbindungen hinsichtlich ihrer Wirkung auf Schädlinge gegen die nachstehenden Verbindungen der DE-A 31 45 448 verglichen:

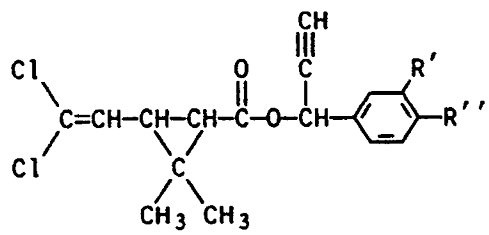

| | | |
|---|---|---|
| I | R′ = $CF_3$ | R′′ = H |
| II | R′ = H | R′′ = $CF_3$ |
| III | R′ = $CH_3$ | R′′ = H |

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens 2 Versuche angesetzt.

Alle geprüften Verbindungen besitzen einen Reinheitsgrad von mindestens 95 %.

Folgende Formulierungen wurden verwendet:

A: Man bereitet eine 0,1 %ige Lösung des Wirkstoffes in Aceton und verdünnt diese Stammlösung entsprechend den angegebenen Dosierungen weiter mit Aceton.

B: Der Wirkstoff wird als Emulsionskonzentrat aufbereitet, indem man ihn in einem Gemisch bestehend aus 70 % Cyclohexanon, 20 % Nekanil LN (ethoxyliertes Rizinusöl) und 10 % Emulphor EL (ethoxyliertes Isooctylphenol). Durch Verdünnen mit entsprechenden Mengen Wasser erhält man Suspensionen mit den angegebenen Wirkstoffkonzentrationen.

A) Blatta orientalis (Schaben), Kontaktwirkung

Der Boden eines 1 l-Glases wird mit der acetonischen Lösung des Wirkstoffes behandelt. Nach Verdunsten des Lösungsmittels setzt man in jedes Glas 5 adulte Schaben. Die Mortalität wird nach 48 Stunden bestimmt.

In diesem Test zeigten die Verbindungen Nr. 31, 41, 44, 56, 60, 61, 66 und 67 eine bessere Wirkung als die Vergleichssubstanz II.

B) Sitophilus granaria (Kornkäfer), Kontaktwirkung

Der Boden von Glaspetrischalen (Ø 10 cm), deren Rand mit Polytetrafluorethylen behandelt ist, damit die Käfer die Schale nicht verlassen können, wird mit 1 $cm^3$ der acetonischen Wirkstofflösung behandelt. Nach Verdunsten des Acetons besetzt man die Schalen mit ca. 50 Kornkäfern. Nach 4 Stunden werden die Käfer in Pappschälchen (Ø 40 mm, Höhe 10 mm) umgesetzt und in die Petrischale zurückgestellt. Die Wirkung bestimmt man nach 24 Stunden in % Mortalität. Käfer die das Pappschälchen nicht mehr verlassen können gelten als tot bzw. schwer geschädigt.

In diesem Test zeigten die Verbindungen Nr. 21, 29, 31, 41, 51, 55, 56, 61, 66, 67 und 72 eine bessere Wirkung als die Vergleichssubstanzen I, II und III.

C) Aedes aegypti (Gelbfiebermücken), Kontaktwirkung

200 ml Leitungswasser in 250 ml Plastikbechern werden mit der wäßrigen Wirkstoffaufbereitung

versetzt und darauf mit 20-30 Moskito-Larven im 3.-4. Larvenstadium besetzt. Die Versuchstemperatur beträgt 25°C. Nach 24 Stunden wird die Wirkung ermittelt.

In diesem Test waren die Verbindungen Nr. 21, 29, 31, 32, 41, 51, 54, 55, 56, 57, 60, 61, 66, 67 und 72 den Vergleichssubstanzen I, II und III überlegen.

D) Musca domestica (Stubenfliegen), Dauerkontakt

Beide Teile einer Petrischale von 10 cm Durchmesser werden mit insgesamt 2 ml der acetonischen Wirkstofflösung behandelt. Nach Verdunsten des Lösungsmittels (ca. 30 Minuten) bringt man je 10 Fliegen in die Schalen. Die Mortalität wird nach 4 Stunden festgestellt.

In diesem Test zeigten die Verbindungen Nr. 21, 29, 31, 32, 41, 43, 44, 51, 54, 55, 56, 57, 60, 66, 67 und 72 eine bessere Wirkung als die Vergleichssubstanzen I und III.

E) Dysdercus intermedius (Baumwollwanzen), Kontaktwirkung

Der Versuch erfolgt in 1 l-Gläsern auf 200 g Quarzsand, der mit 25 ml der wäßrigen Wirkstoffaufbereitung vermischt wurde. Man belegt die Gläser mit ca. 20 Larven von Dysdercus im 3. Larvenstadium und füttert 1x wöchentlich mit eingeweichter Baumwollsaat. Die Kontaktwirkung bonitiert man nach 24 Stunden in % Mortalität.

In diesem Test zeigen die Verbindungen Nr. 31, 32, 41, 43, 44, 51, 54, 55, 56, 57, 60, 61, 66, 67 und 72 eine bessere Wirkung als die Vergleichssubstanzen I, II und III.

F) Plutella maculipennis (Kohlschaben-Raupen), Kontaktwirkung

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird dann mit 10 Raupen des 4. Stadiums belegt. Nach 48 Stunden beurteilt man die Mortalität in Prozent.

In diesem Test zeigten die Verbindungen Nr. 41, 43, 44, 51, 54, 55, 56, 57, 60 und 61 eine bessere Wirkung als die Vergleichssubstanzen II und III.

G) Prodenia litura (Ägypt. Baumwollwurm), Kontaktwirkung

Glaspetrischalen (Ø 10 cm) werden mit der acetonischen Wirkstoffaufbereitung behandelt. Nach dem Verdunsten des Lösungsmittels werden 5 Raupen im 4. Larvenstadium eingesetzt und die Schale verschlossen. Nach 4 Stunden wird die Mortalität festgestellt.

In diesem Test zeigten die Verbindungen Nr. 21, 29, 41, 43, 44, 54, 56, 57, 61, 66 und 67 eine bessere Wirkung als die Vergleichssubstanzen I, II und III.

H) Prodenia litura (Ägypt. Baumwollwurm), Antifeedant-Wirkung

Die Versuche werden in 250 ml Plastikbechern durchgeführt. Verwendet werden je Gefäß 2 Raupen im 3. Larvenstadium, die 24 Stunden keine Nahrung erhielten. Als Versuchssubstrat dienen 1,5 x 2 cm große Backoblaten, die für 5 Sekunden in die wäßrige Wirkstoffaufbereitung getaucht wurden. Nach 24 Stunden ermittelt man die prozentuale Mortalitätsrate.

In diesem Versuch zeigten die Verbindungen Nr. 56, 61 und 66 eine bessere Wirkung als die Vergleichssubstanzen II und III.

**Ansprüche**

1. Substituierte Cyclopropancarbonsäurepropargylester der allgemeinen Formel I

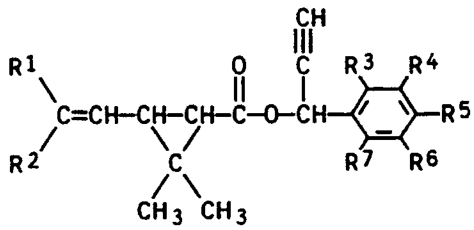

I,

in der die Substituenten die folgende Bedeutung haben:
$R^1$, $R^2$ $C_1$-$C_4$-Halogenalkyl oder Halogen, wobei nicht beide Reste gleichzeitig für Halogen stehen,
$R^3$ bis $R^7$ unabhängig voneinander Wasserstoff, Halogen, Cyano, $C_1$-$C_6$-Alkyl, $C_2$-$C_6$-Alkenyl, $C_2$-$C_6$-Alkinyl, $C_1$-$C_6$-Alkoxy, wobei die Kohlenwasserstoffreste jeweils unsubstituiert oder durch Halogen substituiert sind, oder jeweils zwei benachbarte Reste über eine Gruppe -O-X-O- verbunden sind, wobei
X eine gegebenenfalls halogensubstituierte Methylen- oder Ethylengruppe bedeutet,
mit der Maßgabe, daß $R^4$ nicht Trifluormethyl bedeutet, wenn $R^5$ für ein Chloratom steht.

2. Substituierte Cyclopropancarbonsäurepropargylester der allgemeinen Formel I gemäß Anspruch 1, in der $R^1$ Trifluormethyl und $R^2$ Fluor, Chlor oder Brom bedeuten.

3. Verfahren zur Herstellung substituierter Cyclopropancarbonsäurepropargylester der Formel I gemäß Anspruch 1, dadurch gekennzeichnet, daß man Säuren der allgemeinen Formel II oder Derivate dieser Säuren

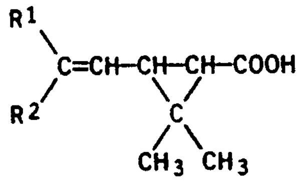

II,

in der die Reste $R^1$ und $R^2$ die in Anspruch 1 angegebenen Bedeutungen haben, mit α-Ethinylbenzylalkoholen der allgemeinen Formel III

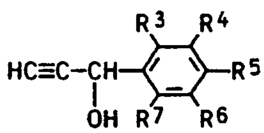

III,

in der $R^3$, $R^4$, $R^5$, $R^6$ und $R^7$ die in Anspruch 1 angegebenen Bedeutungen haben, in an sich bekannter Weise verestert.

4. Schädlingsbekämpfungsmittel, enthaltend substituierte Cyclopropancarbonsäurepropargylester der allgemeinen Formel I gemäß Anspruch 1 und übliche inerte Zusatzstoffe.

5. Schädlingsbekämpfungsmittel nach Anspruch 4, dadurch gekennzeichnet, daß es 0,1 bis 95 Gew.% eines Cyclopropancarbonsäurepropargylesters der Formel I enthält.

6. Verfahren zur Bekämpfung von Schädlingen, dadurch gekennzeichnet, daß man Cyclopropancarbonsäurepropargylesters der Formel I gemäß Anspruch 1 auf die Schädlinge bzw. deren Lebensraum einwirken läßt.

Europäisches Patentamt

# EUROPÄISCHER RECHERCHENBERICHT

Nummer der Anmeldung

EP 90 10 0055

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | PATENT ABSTRACTS OF JAPAN Band 6, Nr. 266 (C-142)(1144), 25. Dezember 1982; & JP - A - 57 158739 (SUMITOMO KAGAKU DOGYO K.K.) 30.09.1982 | 1-4 | C 07 C 69/743<br>A 01 N 53/00<br>C 07 C 67/08 |
| X | EP-A-0 040 742 (BAYER AG) * Anspruch 1 * | 1 | |
| X | DE-A-2 907 609 (MONTEDISON D.P.A.) * Anspruch 1 * | 1 | |
| Y | EP-A-0 121 847 (BAYER AG) * Anspruch 1 * | 1 | |
| Y | EP-A-0 056 154 (BAYER AG) * Anspruch 1 * | 1 | |
| Y | GB-A-2 088 369 (KURARAY CO LTD.) * Anspruch 1 *; & DE - A - 3145448 (Kat. D) | 1 | |
| | | | **RECHERCHIERTE SACHGEBIETE (Int. Cl.5)** |
| A | US-A-4 457 940 (Y. KATSUDA et al.) * Anspruch 1 * | 1 | C 07 C 69/00<br>A 01 N 53/00 |
| A | EP-A-0 009 708 (BAYER AG) * Anspruch 1 * | 1 | |
| A | DD-A- 138 544 (ICI LTD.) * Anspruch 1 * | 1 | |

-/-

Der vorliegende Recherchenbericht wurde für alle Patentansprüche erstellt

| Recherchenort | Abschlußdatum der Recherche | Prüfer |
|---|---|---|
| BERLIN | 26-03-1990 | PROBERT C.L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 90 10 0055

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CHEMICAL ABSTRACTS Band 98, Nr. 7, 14. Februar 1983, Seite 626, Zusammenfassung Nr. 53417w, Columbus, Ohio, US; & JP - A - 57 95944 (KURARAY CO LTD.) 15.06.1982 & CA Formula Index Band 98, Teil 2, Seite 1686F, 3. Spalte, Zeilen 16-20; Seite 1685F, 1. Spalte, Zeilen 96-100 --- | 1 | |
| A | CHEMICAL ABSTRACTS Band 99, Nr. 21, 21. November 1983, Seite 654, Zusammenfassung Nr. 176105w, Columbus, Ohio, US; & JP - A - 58 121246 (KURARAY CO LTD.) 19.07.1983 & CA Formula Index Band 99, Teil 2, Seite 1721F, 3. Spalte, Zeilen 39-43 ----- | 1 | |

RECHERCHIERTE SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| BERLIN | 26-03-1990 | PROBERT C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)